# EUROPEAN PATENT SPECIFICATION

(11) **EP 2 961 290 B1**
(45) Date of publication and mention of the grant of the patent: **16.08.2017**
(21) Application number: 14757802.5
(22) Date of filing: 03.03.2014
(51) Int. Cl.: A41D 13/05, A41D 13/06, A61F 5/01, A63B 71/12

(54) **KNEE BRACE**
KNIESCHIENE
GENOUILLÈRE

(30) Priority: 01.03.2013 US 201361771228 P
(43) Date of publication of application: 06.01.2016
(73) Proprietor: Mueller Sports Medicine, Inc., Prairie du Sac, Wisconsin 53578 (US)
(72) Inventor: MUELLER, Brett, Prairie du Sac, Wisconsin 53578 (US)
(74) Representative: J A Kemp
(86) International application number: PCT/US2014/020036
(87) International publication number: WO 2014/134626

(56) References cited:
- US-A- 5 472 413
- US-B2- 7 749 183
- US-B2- 7 959 590

## Description

### FIELD OF THE INVENTION

This invention relates generally to the field of articles worn by persons to reduce the likelihood, severity, or exacerbation of injury to the body, and more specifically to the field of braces worn on the knee.

### BACKGROUND OF THE INVENTION

Flexible knee braces are used by athletes and other persons engaged in vigorous physical activity to protect the knee from injury and to avoid exacerbation of existing injury. The knee is one of the most heavily used joints of the body, as it is used in any activity that involves walking or running. The knee is also a common subject of injury, due to the relatively high levels of stress it must bear. During normal ambulation, in occupations involving physical labor, and especially during strenuous sports, the knee can undergo abnormal motions as a result of quick changes in direction, fatigue, uneven surfaces, or impacts. These abnormal motions can cause sprains or more serious injuries, including dislocation, stretching, or tearing of the tissues that make up the knee.

Several different types of abnormal motion can cause injury to the knee. First, hyperextension of the knee joint can occur, wherein the knee flexes in its normal front to back fashion but beyond its normal range of motion. A second type of abnormal motion is axial rotation, wherein the lower leg is twisted rotationally relative to the thigh about the knee joint. A third type of abnormal motion is lateral flexure of the lower leg relative to the thigh, wherein the knee joint flexes from side to side instead of the normal front to back motion. In addition, abnormal motion of the patella (kneecap) can result in injuries such as chondromalacia patella, which is a softening or degeneration of the undersurface of the patella, and dislocation of the patella, also known as subluxation of the patella.

Devices to protect the knee against abnormal motions have been used for many years, in a variety of specific embodiments which vary in their abilities to protect against the different types of abnormal motions. Besides protecting the knee against abnormal motions, the devices sometimes provide additional benefits such as insulating the knee to keep it warm, protecting the knee against impact, or compressing the knee to reduce discomfort. However, the protections afforded by these devices against abnormal motion are often accompanied by a reduction in range or ease of normal motion. These devices can also have other undesirable aspects such as added weight on the leg, potential for self-injury or injury to others caused by rigid components, difficulty of application and removal, cost, appearance, and irritation or chafing of the skin.

For these reasons, there has long been motivation to find an improved knee brace which can protect the knee from abnormal motions without affecting the range or ease of normal motion, while avoiding the undesirable aspects of prior art devices.

US 7 959 590 B2 discloses a patella stabilization system for a user's leg. A unitary patella stabilizer sleeve extends at both ends into a plurality of straps for securing the patella stabilizer sleeve around a knee region. An upper inner strap extends from a first upper end of the patella stabilizer sleeve and an upper outer strap extends from a second upper end of the patella stabilizer sleeve, the upper outer strap comprising a fastener at a distal end of the upper outer strap. A lower inner strap extends from a first lower end of the patella stabilizer sleeve and a lower outer strap extends from a second lower end of the patella stabilizer sleeve, the lower outer strap comprising a fastener at a distal end of the lower outer strap. A pair of slots extend substantially non-parallel to a patella opening. A buttress comprising an upper strap and a lower strap is operable to be adjustably positioned on an inside surface of the patella stabilizer sleeve around a location on a circumferential edge of the patella opening and to extend through the pair of slots.

### SUMMARY OF THE INVENTION

According to an aspect of the present invention there is provided the knee brace of claim 1.

Additional aspects of the invention are set out in the dependent claims.

Features and advantages of the invention will be apparent from the following detailed description when taken in conjunction with the accompanying drawings.

### BRIEF DESCRIPTION OF THE DRAWINGS

In the drawings:
Fig. 1A is a plan view of a prior art knee brace, laid flat to expose the exterior surface of the brace;
Fig. 1 B is a plan view of the prior art knee brace of Fig. 1A, laid flat to expose the interior surface of the brace;
Fig. 2A is a plan view of a knee brace according to the present invention, laid flat to expose the exterior surface of the brace;
Fig. 2B is a plan view of the knee brace of Fig. 2A, laid flat to expose the interior surface of the brace;

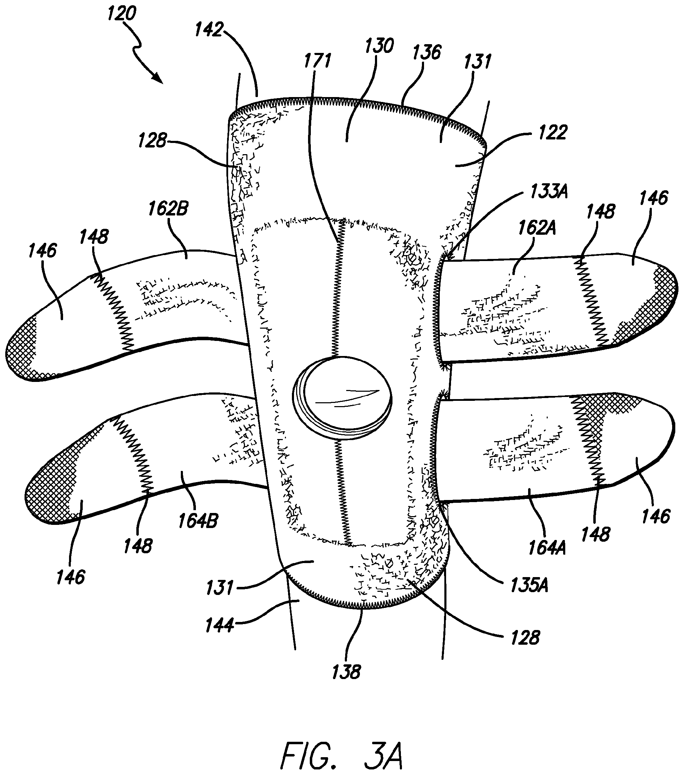

Fig. 3A is a front view of the knee brace of Figs. 2A-2B, applied to the leg of a person with the base mounting straps fastened, but with the spider straps unfastened;

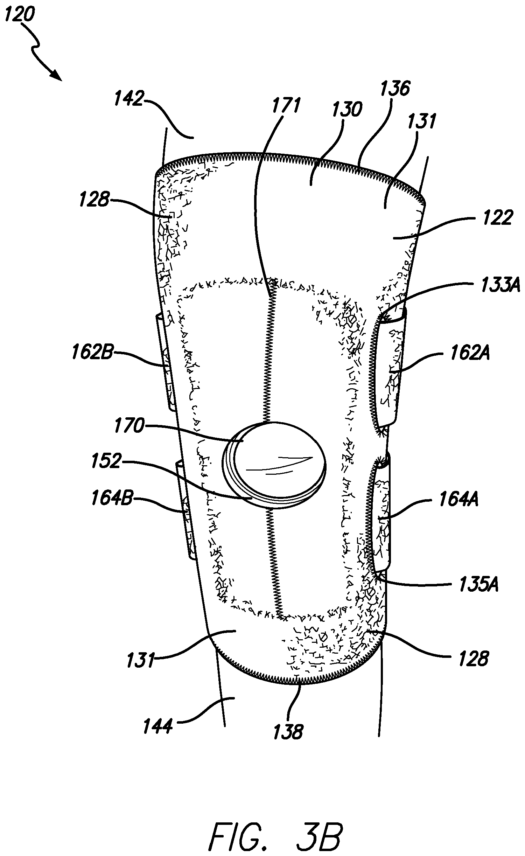

Fig. 3B is a front view of the knee brace of Figs. 2A-2B, applied to the leg of a person with the base mounting straps fastened, and with the spider straps fastened as well;
Fig. 4A is a side view of the knee brace of Figs. 2A-2B, applied to the leg of a person with the base mounting straps fastened, but with the spider straps unfastened; and
Fig. 4B is a side view of the knee brace of Figs. 2A-2B, applied to the leg of a person with the base mounting straps fastened, and with the spider straps fastened as well.

### DETAILED DESCRIPTION OF THE INVENTION

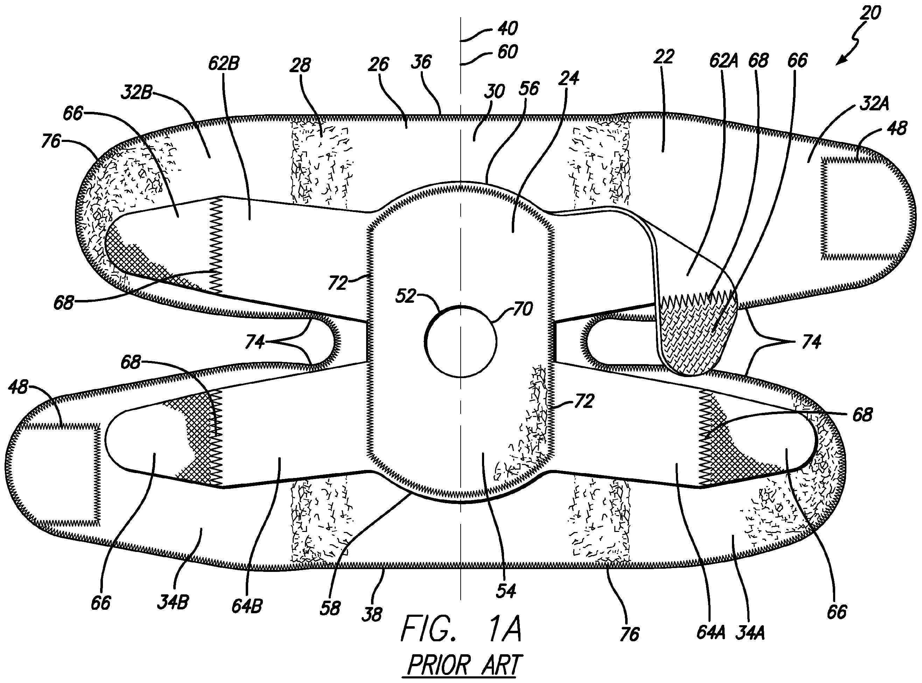

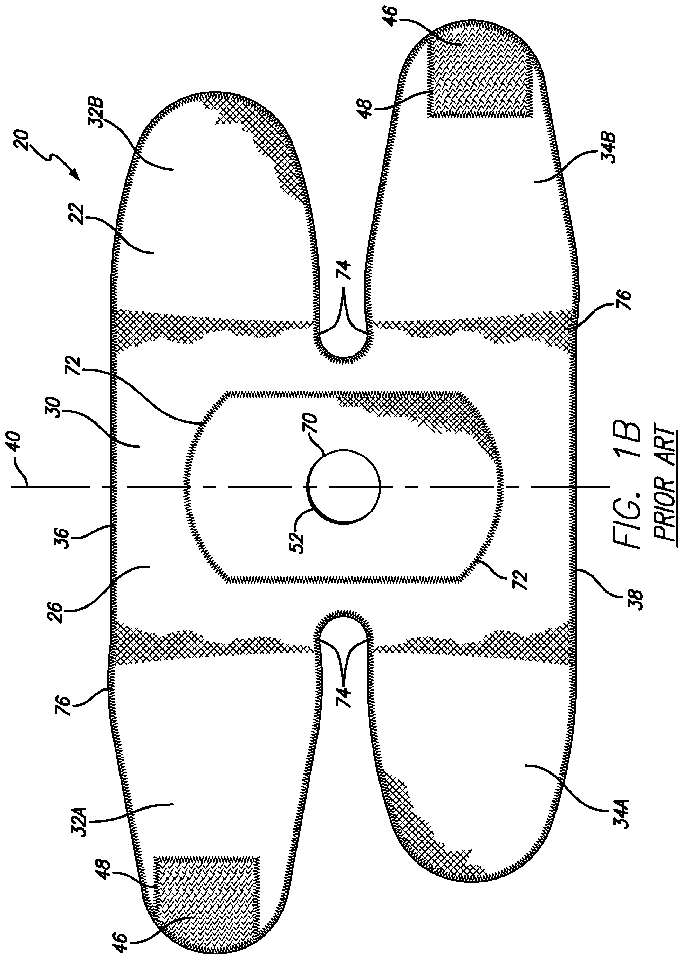

Referring to the drawings, Figs. 1A and 1 B show a prior art knee brace 20, similar to the design taught in US 5 472 413 A. The prior art knee brace 20 includes a base member 22 and a spider member 24, each made by cutting planar sheets 26 of an elastomeric material into the desired shapes. The exterior surface 31 of the base member 22 is preferably covered with
fabric bearing fiber loops 28 that adheres to hook-type material when the fiber loops and hook-type material are pressed together.

The base 22 of the prior art knee brace 20 has a base central portion 30 extending vertically from an upper edge 36 to a lower edge 38, and has a mid-line axis 40 running vertically down the middle of base central portion 30. The base 22 includes a first upper mounting strap 32A, a second upper mounting strap 32B, a first lower mounting strap 34A, and a base second lower mounting strap 34B extending from the central portion 30.

As perhaps best shown in Fig. 1 B which shows the interior surface 39 of the base 22, the first upper mounting strap 32A and first lower mounting strap 34A terminate in hook-type strap fastening tabs 46 suitable for detachable attachment to the fabric bearing fiber loops 28 on the exterior surface 31 of the base member 22. The strap fastening tabs 46 are sewn to the mounting straps with stitches 48.

The base also has a kneecap opening 52 to receive the kneecap when the brace is worn, it may be formed to include a recess 74 to prevent bunching when the brace is worn, and the base preferably includes edge binding 76, although none of these features are required.

As perhaps best shown in Fig. 1A which shows the exterior surface 31 of the base 22, the prior art knee brace 20 includes a spider member 24. The spider member 24 has a spider member central portion 54 extending vertically from an upper edge 56 to a lower edge 58, and has a mid-line axis 60 running vertically down the middle of the spider member central portion 54. The spider member 24 is permanently attached to the exterior surface 31 of the base 22 by stitches 72 that extend around the periphery of the spider member central portion 54.

The spider member 24 includes a first upper tensioning strap 62A, a second upper tensioning strap 62B, a first lower tensioning strap 64A, and a second lower tensioning strap 64B extending from the central portion 54. Each of the tensioning straps 62A, 62B, 64A, 64B terminates in hook-type fastening tabs 66 suitable for detachable attachment to the fabric 28 on the exterior surface of the base 22 and sewn to the tensioning straps with stitches 68. The spider member 24 also has a kneecap opening 70 to receive the kneecap when the brace is worn.

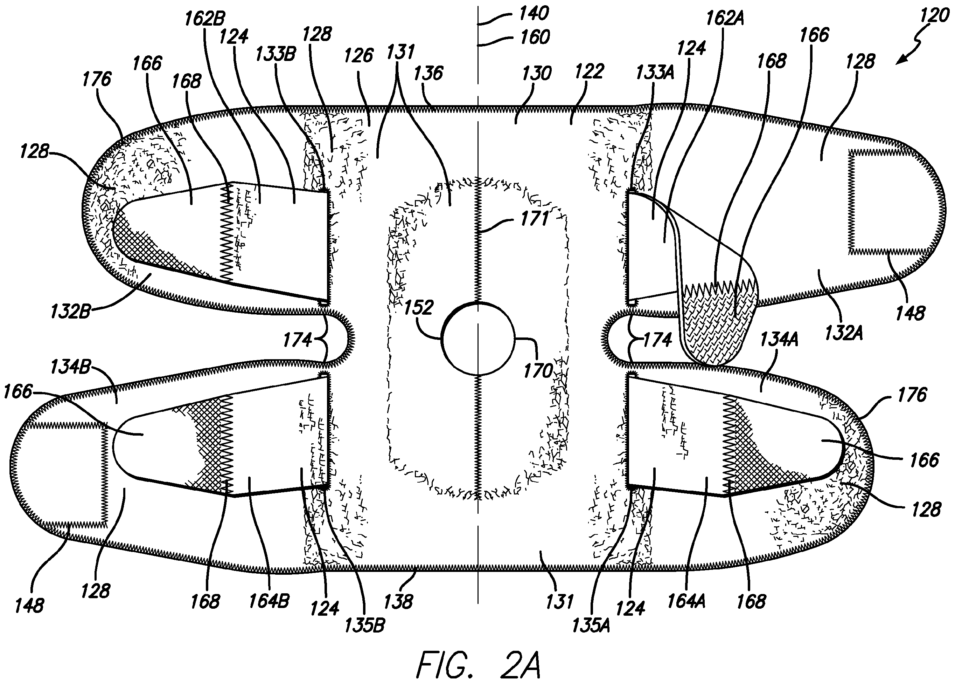

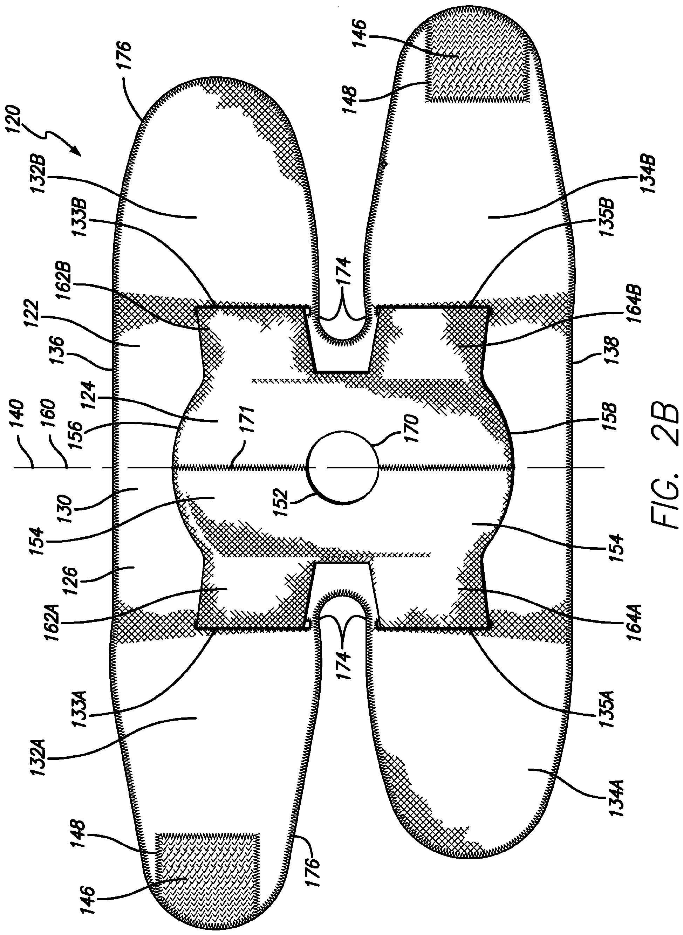

Figs. 2A and 2B show exterior and interior plan views, respectively, of a knee brace 120 according to the present invention laid flat. The knee brace 120 includes a base member 122 and a spider member 124, each made by cutting planar sheets 126 of an elastomeric material into the desired shapes. The outer surface of the base member 122 is preferably covered with fabric bearing fiber loops 128 that adheres to hook-type material when the fiber loops and hook-type material are pressed together.

The base 122 of the knee brace 120 has a base central portion 130 extending vertically from an upper edge 136 to a lower edge 138, and has a mid-line axis 140 running vertically down the middle of base central portion 130. The base 122 includes a first upper mounting strap 132A, a second upper mounting strap 132B, a first lower mounting strap 134A, and a base second lower mounting strap 134B extending from the central portion 130.

As perhaps best shown in Fig. 2B which shows the interior surface 139 of the base 122, the first upper mounting strap 132A and first lower mounting strap 134A terminate in hook-type strap fastening tabs 146 suitable for detachable attachment to the fabric bearing fiber loops 128 on the external surface 131 of the base 122. The hook-type strap fastening tabs 146 are sewn to the mounting straps with stitches 148.

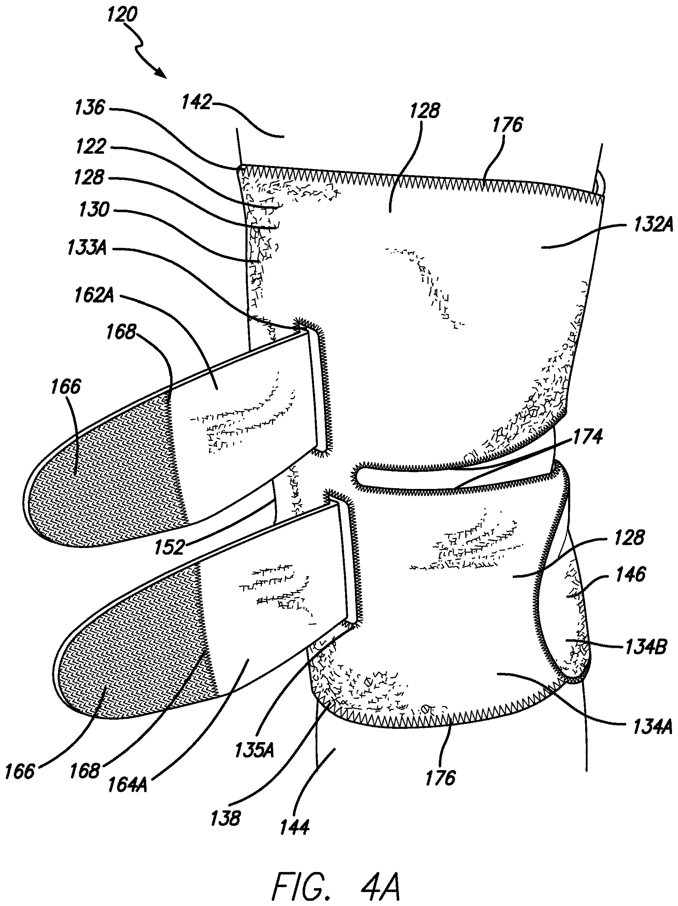

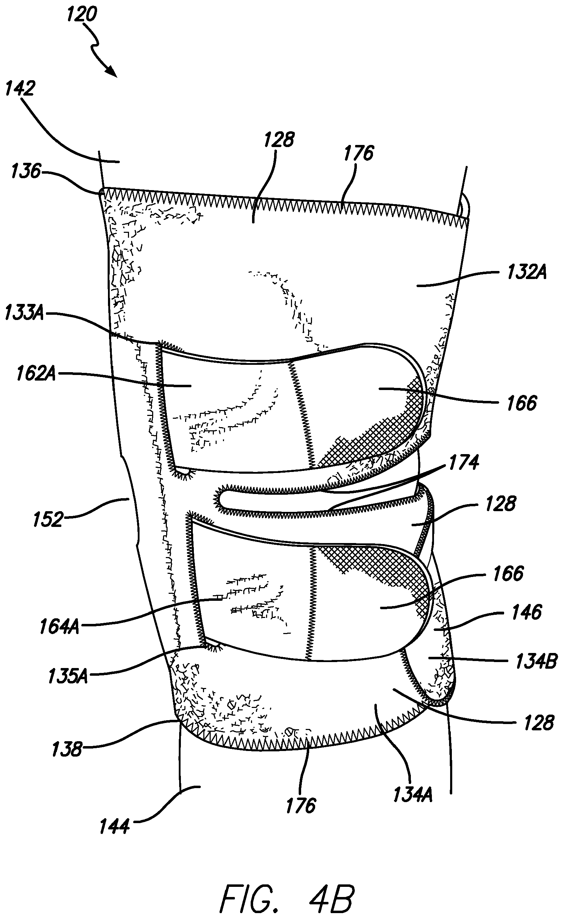

As best shown in Figs. 4A and 4B, when the base 122 of the knee brace 120 is applied to the leg of a person, the first upper mounting strap 132A overlaps the second upper mounting strap 132B at the rear of the leg, allowing the hook-type fastening tab 146 at the end of the first upper mounting strap 132A to adhere to the fabric bearing fiber loops 128 on the exterior surface of the second upper mounting strap 132B in order to fasten the knee brace 120 about the upper leg 142 of the wearer. Similarly, the first lower mounting strap 134A overlaps and adheres to the second lower mounting strap 134B at the rear of the leg in order to fasten the knee brace 120 about the lower leg 144 of the wearer.

The base 122 also preferably has a kneecap opening 152 to receive the patella (kneecap) when the brace is worn. The kneecap opening 152 can match the size of the kneecap, so that the kneecap of the wearer extends from the kneecap opening 152 when the brace 120 is worn, although this is not necessary. The kneecap opening 152 is preferably circular in shape, but this is not necessary and other shapes such as a diamond, oval, rectangle, or square shape may be used. In addition to providing direct patella stabilization, the kneecap opening 152 may help to locate the brace 120 with respect to the kneecap during application of the brace 120.

The base 122 may be formed to include a recess 174 between the upper mounting straps 132A, 132B and the lower mounting straps 134A, 134B, so that when the knee brace 120 is fitted upon the leg the gaps on each side form an opening at the rear of the knee, although this is not required. The recess 174 can help to avoid chafing, it can provide ventilation, and it can help avoid bunching or undue restriction of movement.

The base 122 is preferably formed, as shown in Figs. 2A-2B, as a reclosable sleeve made from a sheet of elastic material that provides generalized support and compression to the knee area, along with therapeutic warming, but other materials may be used. The base 122 may also be formed, for example, as a tubular elastic sleeve shaped to fit snugly about the knee and adjacent leg portions. The base preferably includes edge binding 176, although none of these features are required.

As perhaps best shown in Fig. 2B which shows the interior surface 139 of the base 122, the knee brace 120 includes a spider member 124. The spider member 124 has a spider member central portion 154 extending vertically from an upper edge 156 to a lower edge 158, and has a mid-line axis 160 running vertically down the middle of the spider member central portion 154. The central portion 154 of the spider member 124 is permanently attached to the interior surface 139 of the base 122 by stitches 171 that extend along the mid-line axis 160 of the spider member central portion 54.

The spider member 124 includes a first upper tensioning strap 162A, a second upper tensioning strap 162B, a first lower tensioning strap 164A, and a second lower tensioning strap 164B extending from the central portion 154. Each of the tensioning straps 162A, 162B, 164A, 164B terminates in hook-type fastening tabs 166 suitable for detachable attachment to the fabric bearing fiber loops 128 on the exterior surface of the base 122 and sewn to the tensioning straps with stitches 168. The spider member 124 also has a kneecap opening 170 to receive the kneecap when the brace is worn.

While there are some similarities between the prior art knee brace 20 and a knee brace 120 according to the present invention, there are (without limitation) at least three important differences. First, the spider member 24 of the prior art knee brace 20 is fastened to the exterior surface 31 of the base 22. In contrast, the spider member 124 of the knee brace 120 according to the present invention is fastened to the interior surface 139 of the base 122.

Second, the spider member 24 of the prior art knee brace 20 is fastened to the base 22 by stitches 72 that extend around the periphery of the spider member central portion 54. In contrast, the spider member 124 of the knee brace 120 is fastened to the base 122 by stitches 171 that extend along the mid-line axis 160 of the spider member central portion 54.

Third, both the central portion 54 and the tensioning straps 62A, 62B, 64A, 64B of the spider member 24 of the prior art knee brace 20 are on the exterior surface 31 of the base 22 during normal use. In contrast, in the knee brace 120 the central portion 154 of the spider member 124 is on the interior surface 139 of the base 122, and the tensioning straps 162A, 162B, 164A, and 164B extend through apertures 133A, 133B, 135A, 135B to reach the exterior surface 131 of the base 122.

There are various possibilities with regard to alternative aspects of a knee brace according to the disclosure.

Although in a preferred aspect the knee brace includes a base which is formed as a reclosable sleeve made from a sheet of elastic material, this is not required. For example, the base may also be formed of a tubular elastic sleeve shaped to fit snugly about the knee and adjacent leg portions. The base does not need to include a kneecap opening, and the kneecap opening, if present, could have a variety of shapes, e.g. circular, square, rectangular, elliptical, diamond, trapezoidal, or any substantial equivalent. All such alternative aspects will be referred to herein as a base.

Although in a preferred aspect the lateral sides of the base each terminate in upper and lower fastening straps, with a side recess between the upper and lower fastening straps, this is not required. For example, the sides of the base, or portions thereof, could be straight.

Although in a preferred aspect the base is detachably fastened about the leg of the wearer using hook and loop material of the type which adheres when pressed together, this is not required. For example, other fasteners such as buttons, clasps, buckles, pins, zippers, straps, buttons or other substantial equivalents may be substituted for the hook and loop type fastener material.

Although in a preferred aspect, various components are permanently fastened together using stitches, this is not required. For example, other means such as glue, thermal bonding, or other substantial equivalents could be used.

One or more upright support members may be provided on one side, or on both sides, of the base of the knee brace, to provide support and protect the knee against abnormal motions, although this is not required. The upright support members may be formed, for example, by placing a resilient stay member in an elongated side pocket. The resilient stay members may be comprised of a flattened spiral core of stainless steel or other flexible material of conventional construction commonly used in various types of braces.

The elongate side pocket may be formed, for example, between vertical sewn seams that fix a side pocket cover strip to the base. The side pocket cover strip may be made of the same elastic sheet material as the base, although this is not necessary. Edge binding may be fastened to the edges of the side pocket cover strips, although this is not necessary.

The exact number, location, and construction of the upright support members may vary if provided. For example, there may be a single elongated side pocket forming only one upright support member, or there may be one or more elongated side pockets on each side of the knee with a resilient stay in each elongated side pocket. The elongated side pockets may be openable at one end to allow removal of the resilient stays, so that the brace may be washed or so that different resilient stays may be inserted to adjust the amount and type of support provided. The upright support members may include mechanical hinges, plastic rods, metal rods, narrow strips of reinforcing sheet material, or other substantial equivalents, or a combination of these various alternatives.

Advantageously, the external surface of the front of a knee brace according to the invention does not bear any structure, and can be smooth except for any stitches that secure the spider member to the inside of the base. The smooth external suface can be maintained, for example to provide an attractive and clean appearance that will not snag or obstruct motion during use. Alternatively other structures such as thick knee pads for use in contact sports like football or in trades like concrete or floor tile work, or slippery material for use in sports like volleyball, could be positioned on the external surface for particular applications.

It is understood that the invention is not confined to the embodiments set forth herein as illustrative, but embraces all such forms thereof that come within the scope of the following claims.

## Claims

1. A knee brace (120), comprising:
(a) a base (122) wearable in snug covering relationship to portions of a knee and adjacent portions of a leg of a person, the base having an exterior surface (131) and an interior surface (139) when worn; and
(b) a spider member (124) having a first upper tensioning strap (162A), a second upper tensioning strap (162B), a first lower tensioning strap (164A), and a second lower tensioning strap (164B);
**characterized in that**:
the spider member (124) is positioned between the base (122) and the leg of the person when worn;
the base (122) includes a first upper aperture (133A), a second upper aperture (133B), a first lower aperture (135A), and a second lower aperture (135B); and
the first upper tensioning strap (162A) extends through the first upper aperture (133A), the second upper tensioning strap (162B) extends through the second upper aperture (133B), the first lower tensioning strap (164A) extends through the first lower aperture (135A), and the second lower tensioning strap (164B) extends through the second lower aperture (135B) when the brace is worn.

2. The knee brace (120) of claim 1 wherein at least a portion of the exterior surface (131) of the base (122) bears loop-type material (128), and
wherein each of the first upper tensioning strap (162A), the second upper tensioning strap (162B), the first lower tensioning strap (164A), and the second lower tensioning strap (164B) has a free end bearing a hook-type material (166),
whereby the free ends of the tensioning straps may be detachably attached to the exterior surface of the base.

3. The knee brace (120) of claim 1 wherein the base (122) has a base central portion mid-line axis (140), wherein the spider member (124) has a spider member central portion mid-line axis (160), and wherein the spider member is permanently fastened to the base by a plurality of stitches (171) through at least a portion of the base central portion mid-line axis and through at least a portion of the spider member central portion mid-line axis.

4. The knee brace (120) of claim 3 wherein the spider member (124) is not otherwise permanently attached to the base (122).

5. The knee brace (120) of claim 1 wherein the spider member (124) is permanently fastened to the interior surface (139) of the base (122).

## Patentansprüche

1. Knieschiene (120), umfassend:
(a) eine Basis (122), die in einer eng anliegenden Abdeckbeziehung zu Abschnitten eines Knies und angrenzenden Abschnitten eines Beins einer Person tragbar ist, wobei die Basis beim Tragen eine Außenfläche (131) und eine Innenfläche (139) aufweist; und
(b) ein Kreuzelement (124), das ein erstes oberes Spannband (162A), ein zweites oberes Spannband (162B), ein erstes unteres Spannband (164A) und ein zweites unteres Spannband (164B) aufweist;
**dadurch gekennzeichnet, dass**:
das Kreuzelement (124) beim Tragen zwischen der Basis (122) und dem Bein der Person angeordnet ist;
die Basis (122) eine erste obere Öffnung (133A), eine zweite obere Öffnung (133B), eine erste untere Öffnung (135A) und eine zweite untere Öffnung (135B) enthält; und
das erste obere Spannband (162A) sich beim Tragen der Schiene durch die erste obere Öffnung (133A) erstreckt, das zweite obere Spannband (162B) sich durch die zweite obere Öffnung (133B) erstreckt, das erste untere Spannband (164A) sich durch die erste untere Öffnung (135A) erstreckt und das zweite untere Spannband (164B) sich durch die zweite untere Öffnung (135B) erstreckt.

2. Knieschiene (120) nach Anspruch 1, wobei mindestens ein Abschnitt der Außenfläche (131) der Basis (122) ein schlaufenartiges Material (128) trägt und
wobei jedes des ersten oberen Spannbands (162A), des zweiten oberen Spannbands (162B), des ersten unteren Spannbands (164A) und des zweiten unteren Spannbands (164B) ein freies Ende aufweist, das ein hakenartiges Material (166) trägt,
wobei die freien Enden der Spannbänder abnehmbar an der Außenfläche der Basis angebracht werden können.

3. Knieschiene (120) nach Anspruch 1, wobei die Basis (122) eine Basiszentralabschnitt-Mittellinienachse (140) aufweist, wobei das Kreuzelement (124) eine Kreuzelementzentralabschnitt-Mittellinienachse (160) aufweist, und wobei das Kreuzelement durch eine Vielzahl von Maschen (171) durch mindestens einen Abschnitt der Basiszentralabschnitt-Mittellinienachse und durch mindestens einen Abschnitt der Kreuzelementzentralabschnitt-Mittellinienachse dauerhaft an der Basis befestigt ist.

4. Knieschiene (120) nach Anspruch 3, wobei das Kreuzelement (124) nicht anderweitig dauerhaft an der Basis (122) befestigt ist.

5. Knieschiene (120) nach Anspruch 1, wobei das Kreuzelement (124) dauerhaft an der Innenfläche (139) der Basis (122) befestigt ist.

## Revendications

1. Attelle de genou (120), comprenant :
(a) une base (122) enfilable recouvrant de manière confortable des parties d'un genou et des parties adjacentes d'une jambe d'une personne, la base présentant une surface extérieure (131) et une surface intérieure (139) lorsqu'elle est portée ; et
(b) un élément de croisillon (124) présentant une première sangle de mise en tension supérieure (162A), une deuxième sangle de mise en tension supérieure (162B), une première sangle de mise en tension inférieure (164A), et une deuxième sangle de mise en tension inférieure (164B) ;
**caractérisée en ce que** :
l'élément de croisillon (124) est positionné entre la base (122) et la jambe de la personne lorsque l'attelle est portée ;
la base (122) comprend une première ouverture supérieure (133A), une deuxième ouverture supérieure (133B), une première ouverture inférieure (135A) et une deuxième ouverture inférieure (135B) ; et
la première sangle de mise en tension supérieure (162A) traverse la première ouverture supérieure (133A), la deuxième sangle de mise en tension supérieure (162B) traverse la deuxième ouverture supérieure (133B), la première sangle de mise en tension inférieure (164A) traverse la première ouverture inférieure (135A) et la deuxième sangle de mise en tension inférieure (164B) traverse la deuxième ouverture inférieure (135B) lorsque l'attelle est portée.

2. Attelle de genou (120) selon la revendication 1, dans laquelle au moins une partie de la surface extérieure (131) de la base (122) supporte une matière de type à boucle (128), et
dans laquelle chacune de la première sangle de mise en tension supérieure (162A), la deuxième sangle de mise en tension supérieure (162B), la première sangle de mise en tension inférieure (164A) et la deuxième sangle de mise en tension inférieure (164B) présente une extrémité libre supportant une matière de type à crochet (166),
les extrémités libres des sangles de mise en tension pouvant ainsi être fixées de façon détachable à la surface extérieure de la base.

3. Attelle de genou (120) selon la revendication 1, dans laquelle la base (122) présente un axe de ligne médiane sur la partie centrale de la base (140), l'élément de croisillon (124) présentant un axe de ligne médiane sur la partie centrale de l'élément de croisillon (160), et l'élément de croisillon étant fixé de façon permanente à la base par une pluralité de points de couture (171) par le biais d'au moins une partie de l'axe de ligne médiane de la partie centrale de la base et par le biais d'au moins une partie de l'axe de ligne médiane de la partie centrale de l'élément de croisillon.

4. Attelle de genou (120) selon la revendication 3, dans laquelle l'élément de croisillon (124) n'est pas autrement fixé de façon permanente à la base (122).

5. Attelle de genou (120) selon la revendication 1
dans laquelle l'élément de croisillon (124) est fixé de façon permanente à la surface intérieure (139) de la base (122).
